(19) Europäisches Patentamt
European Patent Office
Office européen des brevets

(11) Veröffentlichungsnummer: **0 000 059**
**B1**

(12)

# EUROPÄISCHE PATENTSCHRIFT

(21) Anmeldenummer: **78100102.9**

(22) Anmeldetag: **06.06.78**

(51) Int. Cl³: **C 07 C 102/08,**
C 07 C 103/34,
C 07 C 103/58,
C 07 C 103/76

(54) **Verfahren zur Herstellung von N-alkylsubstituierten Carbonsäureamiden**

(30) Priorität: **08.06.77 DE 2725889**

(43) Veröffentlichungstag der Anmeldung:
**20.12.78 Patentblatt 78/01**

(45) Bekanntmachung des Hinweises auf die Patenterteilung:
**17.09.80 Patentblatt 80/19**

(84) Benannte Vertragsstaaten:
**BE CH DE FR GB NL**

(56) Entgegenhaltungen:
**US - A - 2 601 387**
**US - A - 3 627 830**

(73) Patentinhaber: **BAYER AG**
**Zentralbereich Patente, Marken und Lizenzen**
**D - 5090 Leverkusen 1, Bayerwerk (DE)**

(72) Erfinder: **Arlt, Dieter, Dr.**
**Rybniker Strasse 2**
**D - 5000 Köln 2 (DE)**
**Behlau, Franz-Gerhard**
**Bromberger Strasse 6**
**D - 4000 Düsseldorf (DE)**

EP 0 000 059 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von N-alkylsubstituierten Carbonsäureamiden

Die Erfindung betrifft ein Verfahren zur Herstellung von N-alkylsubstituierten Carbonsäureamiden.

Es ist bekannt, sekundäre und tertiäre Alkohole, Olefine und Wasser, oder Ester mit Nitrilen in Gegenwart von Säuren, beispielsweise Schwefelsäure, zu N-alkylsubstituierten Carbonsäureamiden umzusetzen (in der Literatur als Ritter-Reaktion bekannt). Zur Aufarbeitung wird das Reaktionsgemisch mit einem großen Überschuß an Wasser behandelt (Russian Chemical Review *29*, 334 (1960)). Hierbei entstehen große Mengen an verdünnten Säuren, die nur mit großen Aufwendungen beseitigt werden können.

Es ist außerdem bekannt (DT—AS 2 144 230), nach Durchführung der Umsetzung durch Extraktion das entstandene Säureamid mit säurestabilen, polaren trisubstituierten Phosphorsäureestern oder tetrasubstituierten Harnstoffderivaten zu extrahieren und damit von Säure zu trennen. Nach der Extraktion wird in bekannter Weise, z.B. durch Destillation, aufgearbeitet.

Es wurde ein Verfahren zur Herstellung von N-substituierten Carbonsäureamiden durch Umsetzung einer Carbonium-Ionen bildenden Komponente mit einem Nitril in Gegenwart von Säuren gefunden, bei dem man die Umsetzung in Gegenwart einer Säure die gegenüber den Umsetzungsprodukten unter Destillationsbedingungen inert ist und die die allgemeine Formel

$$Y—H \qquad (I)$$

worin

Y für einen Acylrest einer Säure aus der Reihe der Phosphorsäuren in der Oxidationsstufe 5 eines Phosphorsäuremono- bzw. diesters und Schwefelsäuremonoesters, einer aliphatischen oder aromatischen Phosphonsäure, einer aliphatischen oder aromatischen Sulfonsäure oder einer aliphatischen Carbonsäure mit einem $p_K$-Wert von größer als 1 steht,

hat, durchführt und dieses Umsetzungsgemisch anschließend durch Destillation auftrennt.

Das erfindungsgemäße Verfahren kann anhand der folgenden Reaktionsgleichung erläutert werden:

$$(CH_3)_3C—OH + C_2H_5CN \quad CH_3SO_3H$$

$$C_2H_5—\overset{O}{C}\,NH—C(CH_3)_3$$

Das erfindungsgemäße Verfahren wird in Gegenwart von Säuren durchgeführt, die unter den Destillationsbedingungen inert sind. Säuren, die für das erfindungsgemäße Verfahren eingesetzt werden können, sollen mit Carbonium-Ionen bildenden Komponenten Carbonium-Ionen bilden und unter den Destillationsbedingungen nicht zu einer Zersetzung des Reaktionsgemisches bzw. des Reaktionsproduktes führen.

Als Acylreste (Y) einer Säure aus der Reihe der Phosphorsäuren mit der Oxidationsstufe 5 seien beispielsweise die Acylreste der Orthophosphorsäure und der Polyphosphorsäure genannt.

Als Acylreste (Y) von Phosphorsäuremono- bzw. -diester und Schwefelsäuremonoester seien Reste der Formel

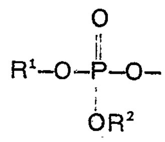 (IIa) oder 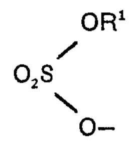 (IIb)

worin

$R^1$ und $R^2$ gleich oder verschieden sind und für Wasserstoff oder einen Alkylrest mit 1 bis 12, bevorzugt 1 bis 8, Kohlenstoffatomen stehen, angeführt.

Beispielsweise seien die folgenden Phosphorsäure- und Schwefelsäureester genannt: Phosphorsäuredimethylester, Phosphorsäurediäthylester, Phosphorsäuremonobutylester, Phosphorsäure-di-2-äthylhexyl-ester, Phosphorsäure-mono-n-butylester, Schwefelsäure-mono-n-butylester, Schwefelsäure-mono-(2-äthyl-hexyl)-ester, Schwefelsäure-mono-(2-hydroxyäthyl)-ester, Phosphorsäure-mono-(2-hydroxyäthyl)-ester.

Außerdem seien als Acylreste der Phosphorsäuremono- bzw. diester und Schwefelsäuremonoester solche von Polyolen, wie Äthan-diol-1,2,Propandiol-1,2, 2,2-Dimethyl-propan-diol-1,3, Trimethylolpropan und Pentaerythrit angeführt.

Als Acylreste (Y) einer aliphatischen oder aromatischen Phosphorsäure seien Reste der Formel

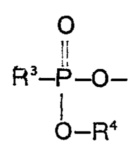 (IIIa) oder 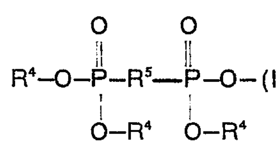 (IIIb)

worin

$R^3$ für einen geradkettigen oder verzweigten Alkyl- oder Alkenylrest, bevorzugt mit 2 bis 4 Kohlenstoffatomen, Aryl, bevorzugt Phenyl, oder Aralkyl, bevorzugt Benzyl,

$R^4$ für Wasserstoff oder einen geradkettigen oder verzweigten Alkylrest, bevorzugt mit 1 bis 4 Kohlenstoffatomen und

$R^5$ für einen gegebenenfalls durch $C_1$ bis $C_4$-Alkyl substituierten Alkylen-, bevorzugt mit 2 bis 6 Kohlenstoffatomen, oder einen Arylenrest, bevorzugt Phenylen, steht, angeführt.

Beispielsweise seien die folgenden aliphatischen oder aromatischen Phosphonsäuren genannt: Methanphosphonsäure, Äthanphosphonsäure, Methanphosphonsäure-monomethylester, Vinylphosphonsäure, Äthan-diphosphonsäure-1,2, Phenylphosphonsäure, Benzyl-phosphonsäure, Benzylphosphonsäuremono-methylester.

Als Acylreste (Y) einer aliphatischen oder aromatischen Sulfonsäure seien Reste der Formel

$$R^6—SO_3— \qquad (IV)$$

worin

$R^6$ für einen gegebenenfalls durch Fluor oder Chlor substituierten Alkylrest oder Aralkylrest, bevorzugt mit 1 bis 18 Kohlenstoffatomen, oder einen gegebenenfalls durch Fluor, Chlor, $C_1—C_4$-Alkyl oder eine Sulfonsäuregruppe substituierten Arylrest, bevorzugt Phenyl, steht, angeführt.

Beispielsweise seien die folgenden Sulfonsäuren genannt: Methansulfonsäure, Äthansulfonsäure, Propansulfonsäure, Butansulfonsäure, Trifluormethansulfonsäure, Perfluorbutansulfonsäure, Benzolsulfonsäure, Benzol-1,3-disulfonsäure, Toluolsulfonsäure, Äthan-di-sulfonsäure-1,2, Butan-di-sulfonsäure-1,4, Benzylsulfonsäure, 4-(2-Dodecyl)-phenylsulfonsäure, Hexadecyl- und Octadecyl-sulfonsäure.

Als Acylreste (Y) einer aliphatischen Carbonsäure mit einem pH-Wert von größer als 1 seien Reste der Formel

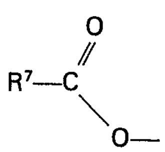
(V)

worin

$R^7$ für einen gegebenenfalls durch Fluor und Chlor substituierten Alkylrest mit 1 bis 4 Kohlenstoffatomen steht, angeführt.

Als Carbonsäuren seien beispielsweise genannt: Dichloressigsäure, Trichloressigsäure, Perchlorpropionsäure, Trifluoressigsäure.

Beispielsweise seien die folgenden für das erfindungsgemäße Verfahren besonders bevorzugten Säuren genannt: Trichlor- und Trifluoressigsäure.

Es ist möglich, das erfindungsgemäße Verfahren in Gegenwart einer oder mehrerer Säuren durchzuführen.

Als Carboniumionen bildende Komponente für das erfindungsgemäße Verfahren seien die Komponenten genannt, die bekanntlich nach der Ritter-Reaktion mit Säuren zu einem Carboniumion aktiviert werden. Beispielsweise sind dies sekundäre oder tertiäre aliphatische Alkohole, Ester und Olefine.

Als sekundäre und tertiäre Alkohole seien Verbindungen der Formel

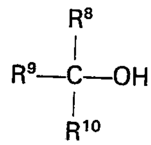
(VI)

worin

$R^8$ Wasserstoff, Alkyl, Aralkyl oder Aryl und

$R^9$ und $R^{10}$ gleich oder verschieden sind und Alkyl oder Aralkyl bedeuten, genannt.

Als Ester seien Verbindungen der Formel

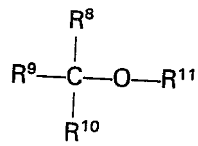
(VII)

worin

$R^8$, $R^9$ und $R^{10}$ die oben genannte Bedeutung haben und

$R^{11}$ einen Acylrest einer anorganischen oder organischen Säure bedeutet, genannt.

Als Olefine seien Verbindungen der Formel

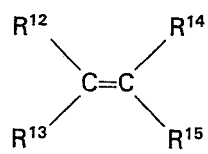
(VIII)

worin

$R^{12}$, $R^{13}$ und $R^{14}$ gleich oder verschieden sind und Wasserstoff, Alkyl, Aralkyl oder Aryl und

$R^{15}$ Alkyl, Aralkyl oder Aryl bedeuten, genannt.

Es ist auch möglich, daß jeweils zwei der Reste $R^{12}$ bis $R^{15}$ zu cyclo-aliphatischen oder aromatischen, carbocyclischen Ringen verbunden sind.

Als Nitrile für das erfindungsgemäße Verfahren seien Verbindungen der Formel

$$R^{16}—CN \qquad (IX)$$

worin

$R^{16}$ Wasserstoff, Alkyl, Alkenyl, Aralkyl oder Aryl bedeutet, genannt.

Alkylreste ($R^8$ bis $R^{10}$, $R^{12}$ bis $R^{16}$) können beispielsweise geradkettige oder verzweigte Kohlenwasserstoffreste mit 1 bis 12 Kohlenstoffatomen sein. Bevorzugt seien genannt: Methyl, Äthyl, Propyl, Isopropyl, Butyl, Isobutyl, Pentyl, Isopentyl, Hexyl und Isohexyl.

Aralkylreste ($R^8$ bis $R^{10}$, $R^{12}$ bis $R^{16}$) können im aliphatischen Teil einen geradkettigen oder verzweigten Kohlenwasserstoffrest mit bis zu 6, bevorzugt mit bis zu 3 Kohlenstoffatomen, und im aromatischen Teil einen Rest aus der Benzolreihe, bevorzugt Phenyl und Naphthyl, haben. Beispielsweise seien genannt: Benzyl, Phenäthyl, Benzhydryl und Phenyl-dimethyl-methyl.

Arylreste ($R^8$ und $R^{12}$ bis $R^{16}$) können aromatische carbocyclische Reste mit 5 bis 8 Kohlenstoffatomen wie Phenyl, Naphthyl und Diphenyl, bevorzugt Phenyl, sein.

Alkenylreste ($R^{16}$) können Vinyl, Propenyl und Cyclopropenyl sein.

Als Säurereste ($R^{11}$) seien beispielsweise der Sulfat-, Phosphat-, Acetat-, Trichloracetat- und der Trifluoracetatrest genannt.

Die Herstellung der einzelnen Komponenten für das erfindungsgemäße Verfahren ist bekannt. Im einzelnen seien beispielhaft die folgenden Verbindungen angeführt:

Als Alkohole: Isopropanol, tert.-Butanol, sek.-Butanol, iso-Amylalkohol, Cyclohexanol, Benzylalkohol, Borneol, Terpineol und Menthol.

Als Ester: Schwefelsäure-mono-ester von Isopropanol, Cyclohexanol, die Trifluoracetate, die bei der Addition von Trifluoressigsäure an Mono- und Diolefine, wie Propen, Isobuten, Cyclopentadien-1,5 und Dicyclopentadien entstehen.

Als Olefine: Propen, Isobuten, Buten - 1, cis- und trans - buten - 2, 2 - Methyl - buten - 2, Diisobuten, Triisobuten, Tetraisobuten, Tri- und Tetrapropen, $\alpha$- und $\beta$-Pinen, Styrol, $\alpha$ - Methyl - styrol, Cyclopenten, Cyclohexen, 1 - Methyl - cyclohexen, Isopren, Dicyclopentadien, 2,5 - Dimethylhexadien - 1,5, Limonen, Methallylchlorid, Methallylcyanid, 3 - Cyclohexen - 1 - carbonsäurenitril, 4 - Methylcyclohexen - 1 - carbonsäurenitril, 3 - Cyclohexen - 1 - carbonsäure - methyl - ester, 3 - Cyclohexen - 1 - carbonsäure - äthyl - ester, 3 - Cyclohexen - 1 - carbonsäure - butyl - ester und Abietinsäure - methylester.

Als Nitrile: Blausäure, Acetonitril, Acrylnitril, Propionitril, Pivalonitril, Laurinsäurenitril, Stearinsäurenitril, Benzylcyanid und Benzonitril.

Das erfindungsgemäße Verfahren wird im allgemeinen im Temperaturbereich von etwa 0°C bis etwa 120°C, bevorzugt von 10°C bis 100°C, besonders bevorzugt von 25° bis 90°C, durchgeführt. Bei Temperaturen die oberhalb 60°C liegen, kann es vorteilhaft sein, die Reaktion unter Druck auszuführen, um unumgesetzte oder überschüssige, niedrig siedende Reaktionskomponenten, wie z.B. Blausäure, in der flüssigen Reaktionsphase zu halten.

Zur Erzielung hoher Raum-Zeit-Ausbeute kann es zweckmäßig sein, das Nitril mindestens stöchiometrisch bezogen auf die Carbonium-Ionen bildende Komponente einzusetzen, vorteilhaft kann ein Überschuß von Nitril gegenüber Carbonium-Ionen bildenden Komponenten bis 10:1 eingesetzt werden, im allgemeinen empfiehlt es sich, das Nitril im molaren Verhältnis von 1 bis 3 gegenüber den Carbonium-Ionen bildenden Komponenten einzusetzen. Günstige Raum-Zeit-Ausbeuten erhält man weiterhin dadurch, daß die molare Menge der Säure mindestens den Wert der Summe der molaren Mengen von bereits im Reaktionsmedium befindlichem Carbonsäureamid und der Carbonium-Ionen bildenden Komponente erreicht. Ein Überschuß der Säure über diesen Wert hinaus erhöht die Reaktionsgeschwindigkeit; im allgemeinen empfiehlt es sich, die Säure gegenüber der Summe der Carbonium-Ionen bildenden Komponente und dem Carbonsäureamid im Verhältnis 1 bis 3 einzusetzen.

In einer besonderen Ausführungsform des erfindungsgemäßen Verfahrens setzt man bei der Umsetzung zur Steigerung der Reaktionsgeschwindigkeit katalytische Mengen von Perfluoralkansulfonsäuren, wie die Perfluorbutan- oder Perfluoroctansulfonsäure oder Perfluoralkancarbonsäuren, wie Trifluoressigsäure zu. Im allgemeinen liegen die Zusätze zwischen 1 und 10 Mol-% der Gesamtsäuremenge.

Die Destillation nach dem erfindungsgemäßen Verfahren kann in üblicher Weise durchgeführt werden. Beispielsweise kann sie in folgenden Destillationsapparaturen durchgeführt werden: Dünnschichtverdampfer, Fallfilmverdampfer und Schlangenrohrverdampfer.

Die Destillation nach dem erfindungsgemäßen Verfahren kann im allgemeinen bei Destillationsumpftemperaturen von 90° bis 180°C, bevorzugt von 100° bis 150°C, und bei einem Druck von 0,1 mbar bis 1 bar, bevorzugt von 0,1 mbar bis 100 mbar, durchgeführt werden.

Nach dem erfindungsgemäßen Verfahren erhält man nach der Destillation das N-alkylsubstituierte Carbonsäureamid und die Säure. Ein gegebenenfalls anfallendes Gemisch aus Carbonsäureamid und Säure kann gegebenenfalls teilweise wieder in die Umsetzung zurückgeführt werden.

Das erfindungsgemäße Verfahren kann sowohl diskontinuierlich als auch kontinuierlich durchgeführt werden.

Das erfindungsgemäße Verfahren kann wie folgt ausgeführt werden:

Die Carbonium-Ionen bildende Komponente und das Nitril werden einem Reaktor, z.B. einen mit Kühl- und Rührvorrichtungen versehenen Kessel oder einer Kesselkaskade, zugeführt, in dem sich ein Gemisch aus der eingesetzten Säure und gegebenenfalls dem Reaktionsprodukt, befindet.

Für den Fall, daß der Siedepunkt des Carbonsäureamids niedriger ist als der der Säure, führt man nach der Umsetzung das Reaktionsgemisch einer, zweckmäßigerweise kontinuierlich arbeitenden, Destillationsapparatur, vorzugsweise einem Dünnschichtverdampfer, einen Fallfilmverdampfer oder einem Schlangenrohrverdampfer, zu und trennt in Abhängigkeit von den Reaktionsbedingungen (z.B. Temperatur, Druck, Verdampferfläche und Verweilzeit) das Carbonsäureamid ganz oder teilweise ab. Der Destillationssumpf, der neben der erfindungsgemäß eingesetzten Säure noch restliches Carbonsäureamid enthält, kann selbstverständlich vollständig durch weitere Destillation aufgetrennt werden. Es ist aber auch möglich, insbesondere bei kontinuierlicher Arbeitsweise, den Destillationssumpf ganz oder teilweise in die Umsetzung zurückzuführen. Um

den Anteil an Nebenprodukten gering zu halten, trennt man vorteilhafterweise 0,1 bis 5% des Destillationssumpfes ab und führt diesen Teil gegebenenfalls einer gesonderten Aufarbeitung, z.B. einer Rückstandsverbrennung zu.

Für den Fall, daß der Siedepunkt der Säure niedriger ist als der des Carbonsäureamids, kann es zweckmäßig sein, die Säure durch eine Trägergas- bzw. Trägerdampfdestillation vom zurückbleibenden Carbonsäureamid zu trennen. Als Trägergase kommen hier beispielsweise Inertgase, wie Helium, Argon und Stickstoff, und als Trägerdampf die eingesetzten Nitrile, wie Blausäure, Acetonitril und Acrylnitril in Frage. Für eine Trägerdampfdestillation können beispielsweise auch Chlorkohlenwasserstoffe, wie Chlor- und Dichlorbenzol, verwendet werden.

Es war nicht zu erwarten, daß sich das bei der Umsetzung erhaltene Gemisch durch Destillation aufarbeiten läßt, da unter diesen Bedingungen eine Zersetzung des Reaktionsproduktes zu erwarten war. Eine solche Zersetzung tritt beispielsweise bei der für die Ritter-Reaktion als Reaktionsmedium besonders oft angewandten Schwefelsäure auf (DT-AS 2 144 230, Spalte 1, Zeile 41 bis Spalte 2, Zeile 20).

Diese Schwierigkeit macht es notwendig, das Reaktionsprodukt z.B. mit einem großen Überschuß an Wasser zu behandeln.

Durch das erfindungsgemäße Verfahren wird vorteilhafterweise auch ein Extraktionsschritt gespart, ohne den der DT-AS 2 144 230 zufolge eine Aufarbeitung durch Destillation nicht möglich ist.

Das erfindungsgemäße Verfahren hat den Vorteil, daß bei der Isolierung des Reaktionsproduktes keine Abfallsäuren oder Abfallsalze anfallen; es läßt sich daher vorteilhaft und ohne Belastung der Umwelt durchführen.

Die nach dem erfindungsgemäßen Verfahren herstellbaren N-substituierten Carbonsäureamide können als Druckereihilfsmittel bzw. bei der Herstellung von Textilhilfsmitteln eingesetzt oder zu Aminen umgesetzt werden.

## Beispiel 1

In eine Lösung von 150 g Blausäure in 420 g Methansulfonsäure, die sich in einem mit Rückflußkühler und Tropftrichter versehenen Rührgefäß befindet, werden unter Rühren und Kühlen 222 g tert.-Butanol innerhalb einer Stunde zudosiert. Die Reaktionstemperatur wird während des Eintropfens langsam ansteigend auf 20 bis 40°C gehalten. Das Reaktionsgemisch wird 4 Stunden bei 40°C gehalten. Dann wird die überschüssige Blausäure im Vakuum abdestilliert. Das zurückbleibende Reaktionsgemisch wird bei einer Verdampferwandtemperatur von 110 bis 115°C in einen Dünnschichtverdampfer eindosiert. Dabei destillieren 55 g eines Gemisches von 96% tert.-Butylformamid und 4% tert.-Butanol (gaschromatographisch ermittelt) ab und es werden 665 g eines Sumpfproduktes aus Methansulfonsäure und tert.-Butylformamid im Verhältnis 64:36 (nach Kernresonanzspektrum) erhalten.

## Beispiel 2

In 200 g Methansulfonsäure, die 1 g Phenothiazin als Polymerisationsinhibitor enthält, wird innerhalb einer Stunde eine Lösung von 150 g tert.-Butanol in 159 g Acrylnitril bei einer Reaktionstemperatur von 30 bis 35°C zugetropft. Das Reaktionsgemisch verbleibt 6 Stunden bei dieser Temperatur im Reaktionsgefäß und wird nach Abdestillieren des überschüssigen Acrylnitril mit 900 g Äthanol vermischt und in einen Schlangenrohrverdampfer eindosiert. Bei 150°C und 25 bar destillieren mit dem Alkohol 100 g N-tert.-Butylacrylamid ab, die durch abdestillieren des Äthanols isoliert werden, Fp. 125°C. Der Sumpf kann mit gleichem Ausbeuteergebnis in die Reaktionsstufe zurückgeführt werden.

## Beispiele 3—23

Das wie in Beispiel 1 jeweils erhaltene Sumpfprodukt (36% tert.-Butylformamid, 64% Methansulfonsäure) wird entsprechend Beispiel 1 mit Blausäure und tert.-Butanol umgesetzt und aufgearbeitet. Die Tabelle gibt die eingesetzten und erhaltenen Produkte an:

| Beispiel Nr. | Eingesetztes Sumpfprodukt (g) | % tert.-Butylformamid | % Methansulfonsäure | Ausgangsstoffe Blausäure (g) | tert.-Butanol (g) | Ausbeute tert.-Butylformamid (g) | (%) |
|---|---|---|---|---|---|---|---|
| 3 | 665 | 36 | 64 | 121,5 | 150 | 204 | 98 |
| 4 | 653 | 35 | 65 | 119,0 | 147 | 205 | 97 |
| 5 | 640 | 33 | 67 | 117,0 | 144 | 200 | 97 |
| 6 | 628 | 33 | 67 | 115,0 | 141 | 190 | 95 |
| 7 | 610 | 35 | 65 | 111,4 | 138 | 175 | 96 |
| 8 | 603 | 34 | 66 | 110,0 | 136 | 175 | 97 |
| 9 | 590 | 34 | 66 | 108,0 | 133 | 170 | 95 |
| 10 | 581 | 36 | 64 | 106,0 | 131 | 164 | 98 |
| 11 | 568 | 34 | 66 | 103,0 | 128 | 170 | 96 |
| 12 | 557 | 33 | 67 | 102,0 | 126 | 174 | 98 |
| 13 | 549 | 34 | 66 | 100,0 | 124 | 158 | 93 |
| 14 | 535 | 36 | 64 | 98,0 | 121 | 150 | 95 |
| 15 | 519 | 33 | 67 | 95,0 | 117 | 155 | 94 |
| 16 | 505 | 34 | 66 | 92,0 | 114 | 150 | 96 |
| 17 | 492 ++) | 33 | 67 | 90.0 | 111 | 150 | 94 |
| 18 | 480 | 34 | 66 | 88.0 | 108 | 138 | 95 |
| 19 | 460 | 33 | 67 | 84.0 | 104 | 140 | 96 |
| 20 | 443 | 33 | 67 | 81,0 | 100 | 134 | 96 |
| 21 | 428 | 34 | 66 | 78.0 | 97 | 125 | 93 |
| 22 | 413 | 36 | 64 | 75,5 | 93 | 115 | 94 |
| 23 | 398 | 35 | 65 | 73,0 | 90 | 120 | 94 |

+) Auswertung des Kernresonanz-Spektrums

++) geringe Mengen an Salz

Beispiel 24

In eine Mischung von 90 g Trifluoressigsäure und 185 g Benzylcyanid werden bei 20°C innerhalb von einer Stunde 44 g tert.-Butanol zugetropft. Das Reaktionsgemisch wird dann 6 Stunden bei 60°C gehalten. Dann wird die Hauptmenge der Trifluoressigsäure unter Normaldruck abdestilliert, ein kleiner Teil im Gemisch mit überschüssigem Benzylcyanid bei vermindertem Druck (Siedebereich bis 75°C/0,5 Torr). Der Destillationssumpf besteht aus nahezu reinem N-tert.-Butyl-phenylacetamid.

Fp. 114°C (aus Alkohol umgelöst), Ausbeute 69 g.

Beispiel 25

In eine Mischung von 163 g Trichloressigsäure und 54 g Adipinsäuredinitril werden bei 80°C 100 g tert.-Butanol innerhalb von einer Stunde zugetropft. Die Reaktionsmischung wird 7 Stunden bei 80°C gehalten. Danach wird das Reaktionsprodukt durch Vakuumdestillation der eingesetzten Trichloressigsäure, die bei ca. 100°C/13 Torr abdestilliert wird, abgetrennt. Man erhält 90 g Adipinsäure-N-tert.-butyl-

diamid, das als fester Rückstand im Reaktionsgefäß zurückbleibt.
Fp. 213 bis 215°C (aus Äthanol umgelöst).

Beispiel 26

Unter Kühlung werden in eine Lösung von 1 g Phenothiazin in 90 g Trifluoressigsäure 84 g Acrylnitril bei 20°C eingetropft. Bei 35°C werden danach 43 g tert.-Butanol zugetropft. Die Reaktionsmischung wird 6 Stunden bei 60 bis 70°C gehalten und anschließend destillativ aufgearbeitet. Acrylnitril, Trifluoressigsäure und ein Teil des entstandenen N-tert.-Butylacrylamids werden unter vermindertem Druck abdestilliert. Zurück bleiben als Destillationssumpf 36 g N-tert.-Butylacrylamid (Fp. 120 bis 122°C). Das Destillat kann mit gleichem Ergebnis wieder in die Reaktion eingesetzt werden.

Beispiel 27

In eine Lösung von 18 g Wasser in 170 g Trifluoressigsäure werden bei Raumtemperatur 126 g Tripropen eingetropft, die gerührte Mischung wird auf 65°C erwärmt und innerhalb von 45 Minuten werden 40 g Blausäure zudosiert. Die Reaktionsmischung wird eine Stunde bei 60 bis 65°C gehalten und anschließend bei einem Druck von 12 bis 15 Torr destillativ aufgearbeitet, wobei die Destillationssumpftemperatur von Raumtemperatur bis auf 149°C gesteigert wird. Man gewinnt auf diese Weise die eingesetzte Trifluoressigsäure und die überschüssige Blausäure und geringe Mengen an N-i-Nonyl-formamid als Destillat vom Siedebereich < 30°C — 139°C/15 Torr, das erneut zur Reaktion eingesetzt werden kann, und anschließend 146 g reines N-i-Nonyl-formamid vom Siedebereich 142 bis 146°C/13 Torr.

Beispiel 28

In eine Mischung von 140 g Polyphosphorsäure (76% $P_2O_5$) und 82 g Acetonitril, die in einem mit Rührer, Tropftrichter und Rückflußkühler versehenen Reaktionsgefäß vorgelegt werden, tropft man bei 50 — 55°C 55 g tert. Butanol ein. Die Reaktionsmischung wird 3 Stunden bei 55 — 60°C gehalten und anschließend in einem Dünnschichtverdampfer eindosiert. Man erhält 77 g Destillat, das anschließend fraktioniert wird, wobei 47 g Acetonitril und 30 g tert. Butylacetamid ($Kp_{15}$ 99 — 100°C, Fp. 93 — 94°C) erhalten werden. Der bei der Dünnschichtdestillation erhaltene Sumpf (185 g) enthält 27% tert. Butylacetamid neben der eingesetzten Polyphosphorsäure, (bestimmt durch Kernresonanzspektroskopie). Dieser Sumpf läßt sich mit gleichem Ausbeuteergebnis in die Reaktion erneut einsetzen.

Beispiel 29

Eine gerührte Mischung von 880 g einer $C_{12}$-Alkyl-benzolsulfonsäure und von 130 ml Blausäure wird unter Rückflußkühlung auf 50°C erwärmt. Dazu werden 181 g tert. Butanol und 70 ml Blausäure zugetropft. Nach 1,5 Stunden wird der Blausäureüberschuß im Vakuum abdestilliert. Bei einer Verdampfertemperatur von 135°C und einem Druck von 0,5 Torr wird das Reaktionsgemisch einer Dünnschichtdestillation unterworfen. Man erhält 101 g tert. Butylformamid und 950 g Destillationssumpf, der erneut zur Reaktion eingesetzt wird. Unter den oben angegebenen Reaktionsbedingungen werden in diesem Sumpfprodukt 80 ml Blausäure mit 75 g tert. Butanol umgesetzt. Die anschließende Dünnschichtdestillation ergibt 110 g tert. Butylformamid.

Beispiel 30

In eine Mischung von 435 g Schwefelsäure-mono-n-butylester und 180 ml Blausäure werden bei 30 — 40°C 259 g tert. Butanol eingetropft, die Reaktion wird innerhalb von 3 Stunden bei 40°C zum Ende geführt. Das Reaktionsgemisch wird einer Dünnschichtdestillation unterworfen. Bei einer Verdampfertemperatur von 115°C und einem Druck von 0,5 Torr werden 90 g Destillat erhalten, das aus einem Gemisch von tert.-Butylformamid, n-Butanol und n-Butylameisensäureester besteht.

Der Destillationssumpf enthält 35% tert. Butylformamid. Unter den angegebenen Bedingungen wird er erneut mit 75 g tert. Butanol und 80 ml HCN umgesetzt. Durch die (wie oben angegeben) anschließende Dünnschichtdestillation erhält man 64 g (94%iges) tert. Butylformamid und 555 Destillationssumpf, der mit gleichem Ergebnis in die Reaktion eingesetzt wurde.

Beispiel 31

In einem Rührgefäß, versehen mit Thermometer, Tropftrichter, Rückflußkühler und Trockenrohr, legt man 660 g p-Dodecylbenzolsulfonsäure vor. Bei 20 — 25°C gibt man 160 ml Blausäure zu. Sobald die Blausäure gut eingerührt ist, erwärmt man auf 30° bis 35°C und tropft innerhalb von 30 min. 148 g tert. Butanol zu. Durch leichtes Kühlen hält man die Temperatur bei 40 — 42°C. Anschließend wird 3 h bei dieser Temperatur nachgerührt, dann die unumgesetzte Blausäure im Vakuum abdestilliert. Man erhält 75 — 80 ml Blausäure zurück.

Der verbleibende Rückstand wird bei einer Wandtemperatur von 145°C in einem Dünnschichtverdampfer eingespeist. Dabei destillieren 106 g eines Gemisches von 94% tert. Butylformamid und 6% tert. Butanol (gaschromatographisch ermittelt) ab. Es werden 750 g Sumpfprodukt erhalten, das die Sulfonsäure und tert. Butylformamid im Verhältnis 2 : 1 (nach Kernresonanzspektroskopie) enthält.

Das Sumpfprodukt läßt sich mit gleichem Ergebnis erneut einsetzen.

**Patentansprüche**

1. Verfahren zur Herstellung von N-substituierten Carbonsäureamiden durch U...-

setzung einer Carbonium-Ionen bildenden Komponente mit einem Nitril in Gegenwart von Säuren, dadurch gekennzeichnet, daß man die Umsetzung in Gegenwart einer Säure, die gegenüber den Umsetzungsprodukten unter Destillationsbedingungen inert ist und die die allgemeine Formel

Y—H

worin

Y für einen Acylrest einer Säure aus der Reihe der Phosphorsäuren in der Oxydationsstufe 5, eines Phosphorsäuremono- bzw. diesters und Schwefelsäuremonoesters, einer aliphatischen oder aromatischen Phosphonsäure, einer aliphatischen oder aromatischen Sulfonsäure oder einer aliphatischen Carbonsäure mit einem $p_k$-Wert von größer als 1 steht, hat, durchführt und dieses Umsetzungsgemisch anschliessend durch Destillation auftrennt.

2. Verfahren nach Anspruch 1, dadurch gekennzeichnet, daß man den Destillationssumpf, der die Säure Y—H und restliches Carbonsäureamid enthält, ganz oder teilweise in die Umsetzung zurückführt, wobei die eingesetzte molare Menge der inerten Säure mindestens den Wert der Summe der molaren Menge von bereits durch die Zurückführung im Reaktionsmedium befindlichen Carbonsäureamid und der Carbonium-Ionen bildenden Komponente erreicht.

3. Verfahren nach Anspruch 1 und 2, dadurch gekennzeichnet, daß die inerte Säure katalytische Mengen von Perfluoralkansulfonsäuren oder Perfluoralkancarbonsäuren enthält.

**Revendications**

1. Procédé pour la préparation d'amides carboxyliques N-substitués par réaction d'un composant formant des ions carbonium avec un nitrile en présence d'acides, ledit procédé etant caractérisé en ce que l'on effectue la réaction en présence d'un acide que est inerte dans les conditions de distillation vis-à-vis des produits de réaction et qui répond à la formule générale

Y—H

dans laquelle Y représente un reste acyloxy d'un acide de la série des acides phosphoriques au degré d'oxydation 5, d'un mono ou diester phosphorique et monoester sulfurique, d'un acide phosphonique aliphatique ou aromatique, d'un acide sulfonique aliphatique ou aromatique ou d'un acide carboxylique aliphatique ayant un pK supérieur à 1, et on sépare ensuite ce mélange de réaction par distillation.

2. Procédé selon la revendication 1, caractérisé en ce que l'on renvoie totalement ou partiellement dans la réaction le résidu de distillation qui contient l'acide Y—H et l'amide carboxylique résiduel, la quantité molaire utilisée de l'acide inerte étant au moins égale à la valeur de la somme de la quantité molaire de l'amide carboxylique se trouvant déjà dans le milieu de réaction par suite du recyclage et du composant formant des ions carbonium.

3. Procédé selon la revendication 1 et 2, caractérisé en ce que l'acide inerte contient des quantités catalytiques d'acides perfluoroalcanesulfoniques ou d'acides perfluoroalcanecarboxyliques.

**Claims**

1. Process for the preparation of N-substituted carboxylic acid amides by reacting a component, which forms carbonium ions, with a nitrile in the presence of acids, characterised in that the reaction is carried out in the presence of an acid which is inert towards the reaction products under distillation conditions and which has the general formula

Y—H

wherein

Y represents an acyloxy radical of an acid from the series of phosphoric acids in the 5th oxidation stage, of a phosphoric acid monoester or diester and sulphuric acid monoester, of an aliphatic or aromatic phosphonic acid, of an aliphatic or aromatic sulphonic acid or of an aliphatic carboxylic acid with a pK value of greater than 1, and this reaction mixture is then separated by distillation.

2. Process according to Claim 1, characterised in that all or part of the distillation sump, which contains the acid Y—H and residual carboxylic acid amide, is recycled to the reaction, wherein the molar amount employed of the inert acid reaches at least the value of the sum of the molar amount of carboxylic acid amide already present, owing to the recycling, in the reaction medium and of the component which forms carbonium ions.

3. Process according to Claim 1 and 2, characterised in that the inert acid contains catalytic amounts of perfluoroalkanesulphonic acids or perfluoroalkanecarboxylic acids.